# EUROPÄISCHE PATENTSCHRIFT

(11) **EP 1 169 068 B1**
(45) Veröffentlichungstag und Bekanntmachung des Hinweises auf die Patenterteilung: **15.10.2003**
(21) Anmeldenummer: 00920531.1
(22) Anmeldetag: 21.03.2000
(51) Int. Cl.: A61L 2/26

(54) **STERILISIERBEHÄLTER**
STERILIZATION CONTAINER
RECIPIENT DE STERILISATION

(30) Priorität: 25.03.1999 DE 19913417
(43) Veröffentlichungstag der Anmeldung: 09.01.2002
(73) Patentinhaber: Wagner, Hans, 80538 München (DE)
(72) Erfinder: WAGNER, Peter, D-82319 Starnberg (DE)
(74) Vertreter: Koch, Günther, Dipl.-Ing.
(86) Internationale Anmeldenummer: EP0002502
(87) Internationale Veröffentlichungsnummer: WO00057930

(56) Entgegenhaltungen:
- DE-A- 4 111 075
- DE-A- 4 111 077

## Beschreibung

Die Erfindung betrifft einen Sterilisierbehälter mit einer Ventilanordnung, die einen Medienaustausch innerhalb eines Sterilisators bis in die Vakuum-Trockenphase hinein ermöglicht und in der letzten Belüftungsphase schließt, so daß der zu diesem Zeitpunkt im Behälterinneren herrschende Unterdruck aufrechterhalten und der Behälter hermetisch verschlossen bleibt, wobei die Ventilanordnung einen dem Strömungsdruck ausgesetzten Ventilkörper aufweist.

Derartige Sterilisierbehälter, wie sie z.B. in der DE 41 11 075 C2 beschrieben sind, haben den Vorteil, daß das Sterilisiergut im Behälter dauerhaft ohne die Gefahr einer Kontamination aufbewahrt werden kann, weil der äußere atmosphärische Druck den Behälter hermetisch geschlossen hält, bis eine willkürliche Belüftung erfolgt. Der Ventilkörper steht unter Federvorspannung, die das Ventil in Offenstellung hält, bis in der letzten Belüftungsphase der Druck im Sterilisator ansteigt und den Ventilkörper auf seinen Ventilsitz drückt, wodurch der zur Zeit des Schließens im Behälter herrschende Unterdruck aufrechterhalten wird.

Bei der Ventilanordnung muß jedoch mit Sicherheit verhindert werden, daß der Ventilkörper durch den einströmenden Dampf vorzeitig auf den Ventilsitz gedrückt wird, weil sonst keine zuverlässige Sterilisation stattfinden und der Behälter unter Umständen sogar implodieren könnte. Es hat sich gezeigt, daß die Federvorspannung des Ventilkörpers in die Öffnungsstellung insbesondere dann nicht zuverlässig ein vorzeitiges Schließen verhindern kann, wenn dieser dem Strömungsmitteldruck ausgesetzte Ventilkörper eine erhebliche Flächenausdehnung besitzt. Die Gefahr eines vorzeitigen Schließens besteht ladungsabhängig durch die hohen Einströmgeschwindigkeiten. Je mehr Sterilisiergut im Behälter befindlich ist, desto mehr Dampfkonsum ist in gleicher Zeit erforderlich. Der Sterilisator ist einem zeitlich geregelten Druckanstieg ausgesetzt, d.h. in der Umgebung des Behälters steigt der Druck konstant an, und er dringt auch in den Behälter ein, wobei eine sofortige Kondensation stattfindet (Phasenübergang gasförmig-flüssig setzt Wärme frei). Entsprechend muß Dampf in den Behälter "nachgeschoben" werden - der aber sofort wieder kondensiert ... solange, bis die durch ständige Kondensation erreichte Erwärmung dazu führt, daß kein Dampf mehr kondensieren kann.
Sterilisiert man also einen leeren Behälter, wird "innen" kaum mehr Dampf benötigt als "außen" (um auf 134° zu kommen): das Ventil wird dann nicht wesentlich mit Strömungsdruck belastet.
Sterilisiert man Ladung, wird innen - je nach Gesamtgewicht und Wärmekapazität der Ladung - wesentlich mehr Dampf "konsumiert": es muß also - in der gleichen, vom Sterilisator vorgegebenen Zeit - wesentlich mehr Dampf durch den Ventilspalt als im leeren Zustand. Bei größeren Ladungen besteht demgemäß die Gefahr des Zuschlagens.

Bei den gattungsgemäßen Sterilisierbehältern erfolgt die Ventilsteuerung zweckmäßigerweise über wenigstens einen Temperatursensor mit Hystereseverhalten. Dieser Temperatursensor kann mit einer Schnappscheibenanordnung ausgerüstet sein, wie dies in der DE 41 11 075 C2 beschrieben ist. Ein solcher Sterilisierbehälter steht nach Öffnung und Entnahme des Sterilgutes sofort wieder für einen erneuten Sterilisiervorgang zur Verfügung, ohne daß eine manuelle Ventilbetätigung erforderlich wäre. Um dies zu gewährleisten, muß der Temperatursensor nach Entnahme des Sterilisierbehälters aus dem Sterilisator automatisch wieder in den Ursprungszustand zurückschalten. Bei Temperatursensoren mit einer Schnappscheibenanordnung erfolgt die Rückschaltung durch das Hystereseverhalten des Thermobimetalls. Die Temperatursensoren mit Schnappscheiben, aber auch andere Temperatursensoren, werden daher so ausgelegt, daß die Rückschaltung in einem Temperaturbereich erfolgt, in dem das vom Sensor gesteuerte Vakuumventil mit Sicherheit geschlossen ist, d.h. die Rückschaltung darf nicht vor Abschluß der Belüftungsphase erfolgen. Bei Schnappscheibenanordnungen wird als Rückschalttemperatur im allgemeinen eine Temperatur von 30 bis 50°C angenommen, d.h. eine Temperatur, die normalerweise erst nach Entnahme aus dem Sterilisator und nach Abkühlung des Sterilisierbehälters auf Raumtemperatur erreicht wird.

Es hat sich jedoch gezeigt, daß diese Schalttemperaturen von vorzugsweise 35 bis 40°C unter gewissen Umständen bereits während einer Trocknungsphase innerhalb des Sterilisators erreicht werden können. Diese vorzeitige Abkühlung kann bei der Sterilisation von schweren Stahlladungen auftreten, weil diese Stahlladungen sehr große Kondensatmengen erzeugen, wodurch die Sensoranordnung mit kondensiertem Dampf benetzt werden kann. Setzt nun eine Vakuumtrocknung ein, erfolgt eine Rückverdampfung von Kondensat mit der Folge eines Energieverbrauchs und einer Abkühlung an jenen Stellen, wo eine Verdampfung der Feuchtigkeit eintritt. Erfolgt eine solche Abkühlung im Bereich der Temperatursensoren, so wird das Ende des Sterilisiervorganges vorgetäuscht, und es kann eine vorzeitige Rückschaltung erfolgen, was eine fehlerhafte Schaltung des Vakuumventils zur Folge hätte.

Der Erfindung liegt daher die Aufgabe zugrunde, ein vorzeitiges Schließen des dem Strömungsdruck ausgesetzten Ventils während des Sterilisiervorganges zu vermeiden und eine zuverlässige Vakuumversiegelung beliebiger Ladungen des Sterilisierbehälters, auch schwerer Stahlladungen, zu gewährleisten.

Gelöst wird die gestellte Aufgabe durch die im Kennzeichnungsteil des Patentanspruchs 1 angegebenen Merkmale.

Durch die Anschlaganordnung wird erreicht, daß auch ein hoher Strömungsdruck das Ventil nicht zuschlagen kann.

Damit der Temperatursensor nicht vorzeitig anspricht, ist gemäß Patentanspruch 2 eine thermische Abschirmung vorgesehen.

Diese thermische Abschirmung ergibt auch eine zuverlässige Abschirmung gegen eine Befeuchtung durch herabtropfendes Kondensat und führt zu einer Isolation, wodurch die vorhandenen Temperatursensoren nahezu ideal der Sattdampfkurve folgen, d.h. es erfolgt eine Abkühlung auf Raumtemperatur im wesentlichen allein durch Konvektion. Der Temperatursensor ist zweckmäßig in das Innere des Gasraumes eines Faltenbalges verlegt. Dies ergibt eine perfekte Abschirmung gegen Befeuchtung von herabtropfendem Kondensat.

Der komplette Temperatursensor ist nach dem Zuschalten gasdicht auch gegen den Balgraum des Faltenbalges abgesperrt.
Im Sensorraum herrscht zu diesem Zeitpunkt die Rücksprungtemperatur von ca. 95°C, so daß innerhalb des Sensorraumes die Temperaturfühler nicht weiter abkühlen können (es kann nichts mehr verdampfen); der Sensorraum selbst "konserviert" eine vergleichsweise hohe Temperatur von 95°C während der gesamten Trocknung. Dadurch wird es möglich, die Vakuumtrocknung beliebig lange in einem beliebig niedrigen Vakuum durchzuführen, ohne daß innerhalb des Sensorraumes vorzeitig die Rücksprungtemperatur erreicht wird. Dadurch wird ein frühzeitiges unerwünschtes Schalten des Sensors zuverlässig verhindert.

Durch die Erfindung wird der Vorteil erreicht, daß jede Ladung des Sterilisators beliebig lange trocknen kann, ohne daß die beschriebene zu frühe Abkühlung der Temperatursensoren zu einer Fehlfunktion führen kann.

In der Sterilisiertechnik arbeitet man mit unterschiedlichen Sterilisiertemperaturen von beispielsweise entweder 120°C oder 134°C. Bisher hat man die Sterilisierbehälter mit Temperatursensoren ausgerüstet, die nur auf eine der beiden gängigen Sterilisiertemperaturen abgestimmt waren und bei denen Fehlfunktionen nicht auszuschließen waren, wenn mit einer nicht angepaßten Dampftemperatur sterilisiert wurde.

Durch die Ventilanordnung gemäß Anspruch 3 werden die gewünschten Schaltvorgänge mit Sicherheit und unabhängig davon durchführt, mit welcher Dampftemperatur sterilisiert wird, wobei sich die konstruktive Ausbildung und Anordnung der Schnappscheiben aus weiteren Unteransprüchen und dem anhand der Zeichnung beschriebenen Ausführungsbeispiel ergeben.

Die erfindungsgemäße Ventilanordnung kann an irgendeiner Stelle im Sterilisierbehälter, d.h. im Deckel oder an den Seitenwänden der Wanne angeordnet werden. Bevorzugt wird das Ventil jedoch am Boden der Wanne an einem tiefsten Punkt derart angeordnet, daß während des Sterilisiervorganges, bei dem das Vakuumventil offen ist, das Kondensatwasser also abfließen kann. Die Ableitung von Kondensat ermöglicht somit eine Trocknung des Behälterinhaltes ohne die Notwendigkeit energieverzehrender Rückverdampfung des Kondensats. Erst dadurch wird es möglich, Sterilisierbehälter herzustellen, ohne dabei auf Wärmeleitfähigkeit oder Wärmekapazität des verwendeten Werkstoffs Rücksicht nehmen zu müssen, so daß z.B. auch Behälter aus Kunststoff Anwendung finden können.

Nachstehend werden Ausführungsbeispiele der Erfindung anhand der Zeichnung beschrieben. In der Zeichnung zeigen:
- Fig. 1: ist ein Vertikalschnitt eines erfindungsgemäß ausgebildeten Sterilisierbehälters;
- Fig. 2: ist eine Ansicht der Wanne des Sterilisierbehälters nach Fig. 1 (im rechten Teil von oben her betrachtet, im linken Teil von unten her betrachtet);
- Fig. 3: ist in größerem Maßstab gezeichnet die in Fig. 1 mit X bezeichnete Einzelheit der Deckeldichtung;
- Fig. 4: ist eine Schnittansicht des in den Boden der Wanne eingesetzten Ventils in Offenstellung;
- Fig. 5: im größeren Maßstab gezeichnet eine Schnittansicht der Ventilsteuervorrichtung in der Stellung gemäß Fig. 4;
- Fig. 6: ist eine perspektivische Ansicht der Steuerglieder tragenden Deckplatte der Ventilsteuervorrichtung;
- Fig. 7: ist eine perspektivische Ansicht der Auslaßdichtung der Ventilsteuervorrichtung;
- Fig. 8: ist eine Ansicht des in Offenstellung gegen einen Anschlag überführten Ventilkörpers;
- Fig. 9: ist in größerem Maßstab eine der Fig. 8 entsprechende Ansicht;
- Fig. 10: ist eine Ansicht der Ventilsteuervorrichtung, die diese nach Temperaturanstieg auf eine erste Temperatur (von z.B. 115°C) einnimmt;
- Fig. 11: ist eine Schnittansicht der Ventilsteuervorrichtung, die diese bei Temperaturanstieg nach Erreichen einer zweiten Temperatur (von z.B. 117°C) einnimmt;
- Fig. 12: ist eine Schnittansicht der Ventilsteuervorrichtung nach einer Abkühlungsphase;
- Fig. 13: ist eine Schnittansicht des Ventils in Schließstellung;
- Fig. 14: ist eine Schnittansicht einer zweiten Ausführungsform der Ventilsteuervorrichtung in einer Betriebsstellung gemäß Fig. 5;
- Fig. 15: ist eine Schnittansicht der Ventilsteuervorrichtung gemäß Fig. 14 in Ventilschließstellung gemäß Fig. 13.

Der Sterilisierbehälter besteht aus der Wanne 10 und dem Deckel 12. Die Wanne weist einen Gefälleboden 14 und einen umlaufenden Standrahmen 16 auf. Zwischen dem oberen Rand der Wanne 10 und dem Deckel 12 ist ein im Querschnitt L-förmiger Dichtungsring 18 angeordnet. Dieser Dichtungsring ist mit seinem nach innen weisenden Schenkel in eine umlaufende Nut 20 des Deckels 12 unverlierbar eingesetzt und bewirkt eine Abdichtung nach unten und außen. Der Dichtungsring 18 hat demgemäß einen Formschluß in der Horizontalen, so daß nicht geklebt werden muß, sondern ohne jeden Aufwand vor Ort die Dichtung gewechselt werden kann. Selbst wenn die Dichtung nach langer Lagerung leicht an der Wanne "anklebt", erlaubt der Formschluß das Abnehmen des Deckels, ohne daß die Dichtung sich von diesem löst. Die Dichtung hat einen doppelten Dichtsitz: einerseits (speziell anfangs - wenn der Behälter nicht unter Vakuum steht, sondern nur mit den Verschlüssen verschlossen ist) eine stirnseitige Auflage, aber bei Erhöhung der Anpreßkraft (bei sich aufbauendem Differenzdruck) eine gezielte Verdrängung der Dichtung in den umlaufend eingeschlossenen Hohlraum derart, daß auch die vertikalen Flächen dicht werden.

Die Wanne 10 weist im tief gelegenen Mittelabschnitt Perforationslöcher 22 auf, die dem Medienaustausch und dem Ablauf des Kondensats dienen. In diesem Mittelbereich des Behälterbodens 14 ist eine Ventilanordnung 24 vorgesehen, die einen Dichtring 26 aufweist, der mit einem ringförmigen Ventilsitz 28 zusammenwirkt, welcher vom Boden der Wanne gebildet wird und außerhalb der Perforationslöcher 22 liegt, so daß nach Schließen des Ventils 26, 28 der Behälterinhalt gegenüber der äußeren Atmosphäre abgedichtet ist. Der Dichtring 26 wird von einer dem Strömungsdruck ausgesetzten Ventilplatte 50 getragen und bildet zusammen mit dieser den Ventilkörper.

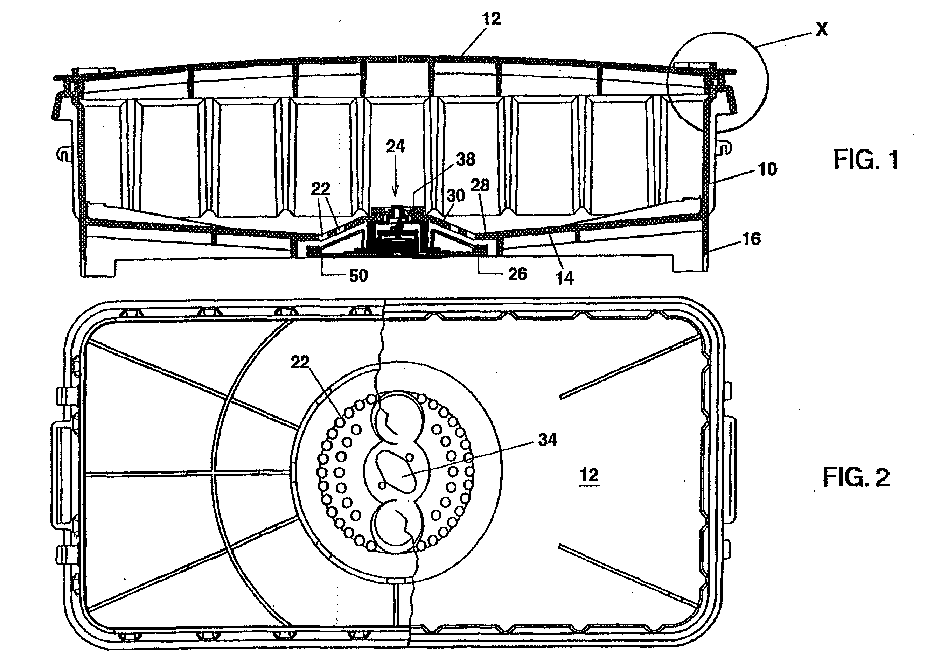

Der Wannenboden 14 weist, wie aus Fig. 1 ersichtlich, im Bereich der Ventilanordnung 24 einen konischen Wandabschnitt 30 mit den Perforationslöchern 22 auf. Er ist derart ausgebildet, daß die Ventilanordnung über der Standfläche des Standrahmens 16 untergebracht werden kann. Im zentralen flachen Abschnitt 32 ist ein Loch 34 ausgebildet, in das von unten her ein Ventildeckel 36 eingesetzt ist, der aus einem rotationssymmetrischen Edelstahlteil besteht und mittels eines Drehverschlusses 38 und mittels eines Seegerringes 40 an dem Wandabschnitt 32 mechanisch festgelegt ist. Der Drehverschluß 38 ist ein oval geformter drehbarer Verschluß, der mittels des Seegerringes 40 am Ventildeckel gehaltert ist. Nach Verdrehen des Drehverschlusses 38 um 90° wird der Ventildeckel 36 am Wannenboden arretiert. Die Einrastung erfolgt durch erhöhte Knöpfe 42. Ein unter dem flachen Abschnitt 32 verlaufender Ventildeckelflansch 44 ist unten umlaufend gasdicht mit einem axial komprimierbaren Well- oder Faltenbalg 46 verschweißt. Ein nach außen gerichteter Ringflansch 48 am unteren Ende des Faltenbalges 46 ist mit der aus Edelstahl bestehenden Ventilplatte 50 über einen Zwischenring 52 verschweißt. Die Ventilplatte 50 trägt an ihrem äußeren Umfang den Dichtring 26. Ein unter den Perforationslöchern 22 konisch nach außen und unten verlaufendes Leitblech 54 ist auf die Ventilplatte aufgesetzt, damit aus den Perforationslöchern 22 heraustropfendes Kondensat nach außen abgeleitet wird und sich nicht auf der Ventilplatte 50 ansammelt. Der Ventildichtring 26 besteht aus einem Elastomer, z.B. Silikon und ist auf der Ventilplatte 50 aufgeklebt oder aufvulkanisiert.

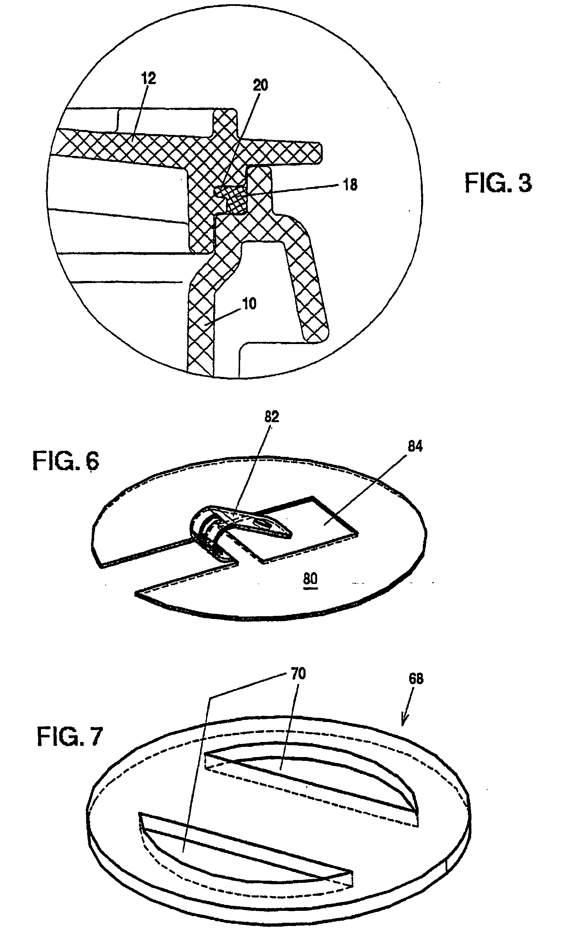

Dieser Dichtungsring könnte auch am Ventilsitz 28 am Boden der Wanne aufgebracht sein. Die Ventilplatte 50 weist eine Entlüftungsöffnung 56 auf, die durch eine Dichtungsscheibe 58 verschlossen ist, welche durch eine an der Ventilplatte 50 angeschweißte Blattfeder 60 dichtend angedrückt wird. Diese Teile wirken als Rückschlagventil, das eine Entlüftung des Ventilinnenraums, d.h. des Raumes innerhalb des Faltenbalgs 46, ermöglicht. Innerhalb des vom Faltenbalg 46 umschlossenen Raumes ist auf der Ventilplatte 50 ein mit Innengewinde versehener Napf 62 aufgeschweißt. Der Boden dieses Napfes 62 weist eine Mittelöffnung 64 auf, die mit einer Belüftungsöffnung 66 in der Ventilplatte 50 fluchtet. Diese Belüftungsöffnung 64, 66 ist durch eine Auslaßdichtungsscheibe 68 abgedeckt, die auf dem Boden des Napfes 62 liegt und gemäß Fig. 7 ausgebildet ist. Sie weist zwei Öffnungen in Form von Kreisabschnitten auf, zwischen denen der die Belüftungsöffnung 64, 66 abdeckende Steg liegt.

In das Innengewinde des Napfes 62 ist die mit Außengewinde versehene Ringwand 72 eines Deckels 74 eingeschraubt. Die untere Stirnringfläche der Ringwand 72 wird dichtend gegen den Rand der Auslaßdichtungsscheibe 68 verspannt. Diese Dichtungsscheibe 68 hat demgemäß eine Doppelfunktion, indem sie einerseits die Belüftungsöffnung 64, 66 bei innerem Überdruck im Ventil absperrt und andererseits eine Dichtung der miteinander verschraubten Teile 62, 72 bewirkt.

Der Deckel 74 besitzt ein Mittelloch 76 mit Einstich für einen Dichtungsring 78. Über diese Mittelöffnung erfolgt die Belüftung des Ventilinnenraums. Auf dem Deckel 74 ist eine Federstahlplatte 80 aufgeschweißt, aus der eine Blockadefeder 82 ausgestanzt und in der aus Fig. 6 ersichtlichen Weise derart umgebogen ist, daß sie einen Ausschnitt 84 der Scheibe 80 übergreift, der über der Mittelöffnung 76 des Deckels liegt. Diese als Blattfeder ausgebildete Blockadefeder 82 trägt einen V-förmig gekerbten Blockadestift 86, der das Zuschlagen des Ventils durch Strömungsdruck verhindert, wie dies weiter unten noch ausführlich beschrieben wird. Der Blockadestift 86 ist mit der Blattfeder 82 über einen Einpreßpin 88 verbunden, der nach unten in die Mittelöffnung 76 einsteht und mit einer polierten Ventilkugel 90 zusammenwirkt, deren Ventilsitz durch den Dichtungsring 78 gebildet wird.

Das von Napf 62 und Deckel 74 gebildete, den Temperaturfühler umschließende Gehäuse bildet, zusammen mit dem Ventildeckel 36 und dem Faltenbalg 46, eine thermische Abschirmung für den Temperaturfühler und verhindert ein Eindringen von herabtropfendem Kondensat und damit eine vorzeitige Schaltung des Schnappscheibentemperatursensors. Dieser könnte mit nur einem Schnappscheibentyp realisiert werden, der das Ventil offenhält, bei Erreichen der Stelltemperatur "heiß" das Ventil verschließt (Entlüftung über das Rückschlagventil), und dann erst - geschützt durch den isolierten Einbau - nach erfolgter Versiegelung (Vakuum) und Abkühlung des Behälters wieder öffnet (um das Ventil auf die nächste Verwendung vorzubereiten).

Denkbar wären z.B. folgende Schalttemperaturen des Temperatursensors:
- 134°C bei Erwärmung / 30-50°C bei Abkühlung: dieses Ventil würde in jedem Sterilisationsprogramm "funktionieren", das 134°C erreicht.
   Nachteil: Es würde aber nicht schalten, wenn z.B. ein 120°C- Programm gefahren wird (da es ja dann nie "ZU"-schalten würde.
- 120°C bei Erwärmen / 30-50°C bei Abkühlung: dieses Ventil würde in einem 120°C-Programm funktionieren, und bedingt auch in einem 134°C-Programm - jedoch mit Risiken verbunden:
   wenn das Ventil bei 120°C schließt, ist ein weiterer Druckanstieg im Gasraum des Faltenbalges nicht mehr möglich (nur noch dessen Entlüftung ...): bei 120°C herrscht (Sattdampfkurve) aber erst ein Druck von 2,1 hPa. Wird ein Behälter nun in einem 134°C-Programm sterlisiert, erfolgt ein weiterer Druckanstieg auf 3,2 - 3,4 hPa. Dieser Druckanstieg (Differenz ist 1,1 - 1,3 hPa) würde den Faltenbalg zusammendrücken, könnte also nicht in den Behälter eindringen - mit dem Ergebnis, daß entweder innerhalb des Behälters die korrekten Sterilisationsbedingungen nicht erreicht werden, oder daß der Behälter die Druckdifferenz nicht aushält und implodiert.

134°C- und 120°C-Programme sind die beiden Standard-Temperaturniveaus bei der Krankenhaussterilisation. Ein Anwender müßte also über verschiedene Ventile verfügen (für 120°C- oder 134°C-Niveau) und diese vor Verwendung auch anbringen bzw. austauschen. Das ist denkbar, aber umständlich und fehleranfällig. Die nachstehend beschriebene Schnappscheibenanordnung vermeidet diesen Nachteil, indem das Ventil so gestaltetwird, daß es bei allen gängigen Sterilisationsniveaus verwendet werden kann.

Die Ventilkugel 90 wird von einer kalottenförmig nach oben konvex verlaufenden Edelstahlscheibe getragen, mit der sie verschweißt ist. Diese Edelstahlscheibe 92 erhält ihre Form unabhängig von Temperatur- und Druckänderungen bei und verschiebt sich lediglich innerhalb des durch die Ringwand 72 begrenzten Raumes, indem sie mit den nachstehend beschriebenen Schnappscheiben zusammenwirkt. Diese Schnappscheiben bestehen aus Thermobimetall und sind dadurch gekennzeichnet, daß sie bei einer vorbestimmten Erwärmungstemperatur in ihren entgegengesetzten Wölbungszustand umschalten und bei Abkühlung mit einer Hysterese behaftet bei einer niedrigeren Schalttemperatur zurückschnappen. In Fig. 5 sind die nachstehend beschriebenen Schnappscheiben in ihrem Wölbungszustand gezeichnet, den sie bei Raumtemperatur einnehmen. Die der Edelstahlscheibe 92 benachbarte Schnappscheibe 94 ist entgegen der Stahlscheibe 92 gewölbt und nach oben konkav. Diese Schnappscheibe 94 hat typischerweise die folgenden Umschalttemperaturen:

Bei Erwärmung erfolgt bei 115°C ein Umschnappen von der konkaven Lage in die konvexe Lage. Beim Abkühlen erfolgt ein Rückschnappen von der konvexen in die konkave Lage bei 95°C.

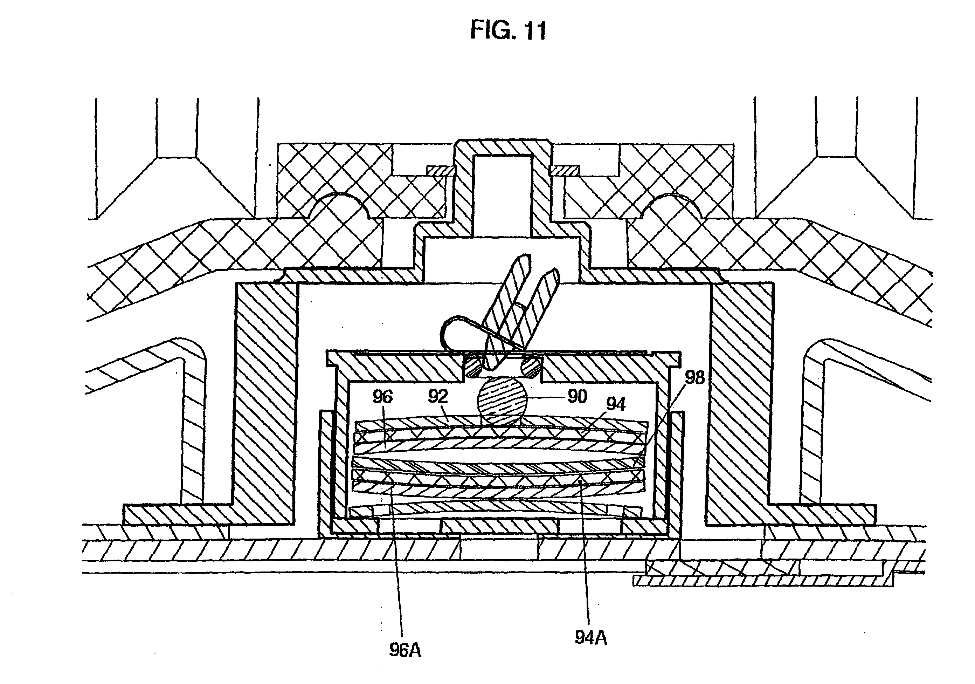

Der Schnappscheibe 94 benachbart liegt eine weitere Schnappscheibe 96 mit einer gegenüber der Schaltcharakteristik der Schnappscheibe 94 anderen Schaltcharakteristik. Die Einbaulage der Schnappscheibe 96 entspricht jener der Schnappscheibe 94, und sie liegt nach oben konvex der Schnappscheibe 94 vollflächig an, wenn die Temperatur 117°C überschreitet (Fig. 11) . Die Thermobimetall-Schnappscheibe 96 hat typischerweise die folgenden Schalttemperaturen: Bei Erwärmung erfolgt ein Umschnappen von der konkaven Lage in die konvexe Lage bei 117°C. Beim Abkühlen erfolgt ein Rückschnappen von der konvexen Lage in die konkave Lage bei 35 bis 50°C.

Benachbart zu der Schnappscheibe 96 liegt eine weitere Edelstahlscheibe 98, die nach oben konkav ausgebildet ist und der Wölbung der eingesetzten Schnappscheiben entspricht; sie ändert ihre Form nicht, sondern nur ihre Lage.

Benachbart zur Edelstahlscheibe 98 liegt eine nach oben konvex gekrümmte Schnappscheibe 94A mit gleicher Schaltcharakteristik wie die Schnappscheibe 94. Benachbart zu dieser Schnappscheibe 94A liegt eine ebenfalls nach oben konvex gewölbte Schnappscheibe 96A, die die gleiche Schaltcharakteristik besitzt wie die Schnappscheibe 96. Die Schnappscheibe 96A wird von einer weiteren formbeständigen Edelstahlscheibe 99 abgestüzt, die nach oben konvex gewölbt ist und in ihrer Wölbung genau der Wölbung der Schnappscheiben entspricht. Diese Edelstahlscheibe ist mit Löchern zur Verbesserung des Dampfeinlasses versehen und am Umfangsrand auf der Äuslaßdichtungsscheibe 68 abgestüzt. Der Zwischenring 52 bildet eine Beilagescheibe zur Schaffung einer Distanz zwischen dem Ringflansch 48 des Faltenbalges 46 und der Ventilplatte 50 und ist mit diesen Teilen jeweils gasdicht durch Verschweißung, Verklebung oder Verschraubung verbunden.

In der Zeichnung ist der Übersichtlichkeit wegen jeweils nur die die Funktion bestimmende Schnappscheibe dargestellt. In der Praxis kann es zweckmäßig sein, jeweils mehrere gleiche und gleichgerichtet eingebaute Schnappscheiben zu benutzen.

Die Funktion der Ventilanordnung 24 wird im folgenden anhand der Fig. 4 bis 13 beschrieben:

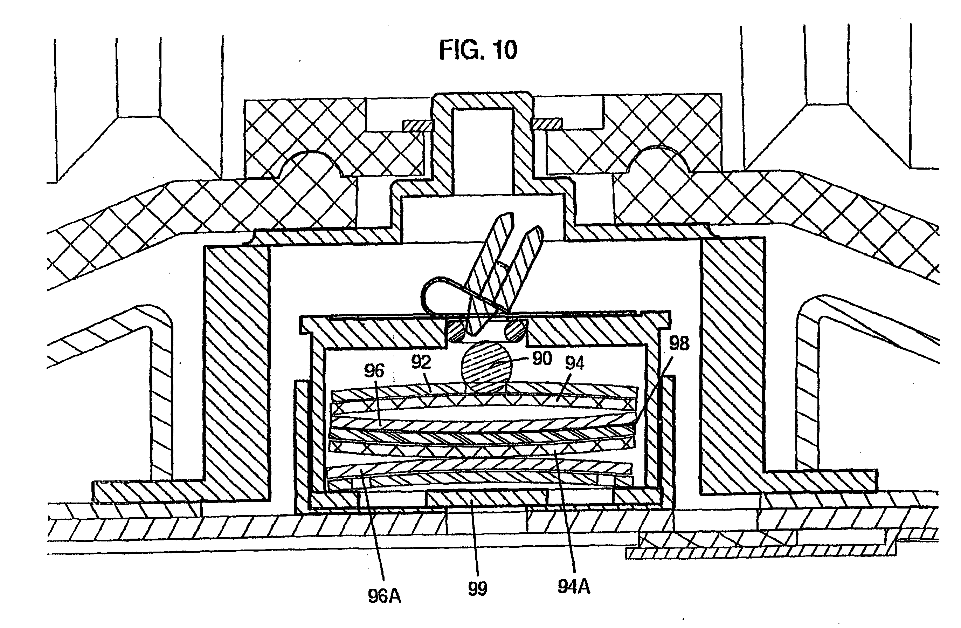

Die in den Fig. 4 und 5 dargestellte Stellung der Schnappscheiben bleibt bei Erwärmung bis auf 115°C erhalten. Bei 115°C schalten die Schnappscheiben 94 und 94A in ihre entgegengesetzte Wölbungsstellung gemäß Fig. 10 um. Die Gesamthöhe und damit die Lage der Ventilkugel 90 ändert sich dabei nicht.

Bei Erreichen der zweiten Umschalttemperatur von 117°C schalten zusätzlich die Schnappscheiben 96 und 96A in ihre entgegengesetzte Wölbungsstellung um, wie dies aus Fig. 11 ersichtlich ist. Die Gesamthöhe des Stapels und die Lage der Ventilkugel 90 bleibt unverändert, so daß der Ventilring 26 von seinem Ventilsitz 28 abgehoben bleibt, d.h. das Ventil bleibt offen, so daß der Medienaustausch ungehindert weiter erfolgen kann.

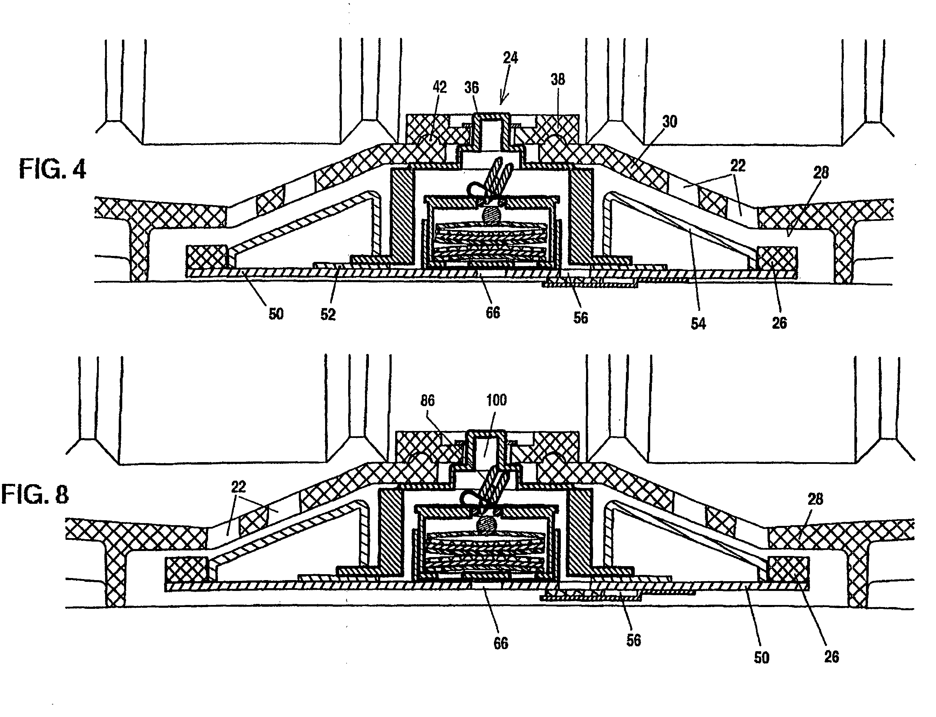

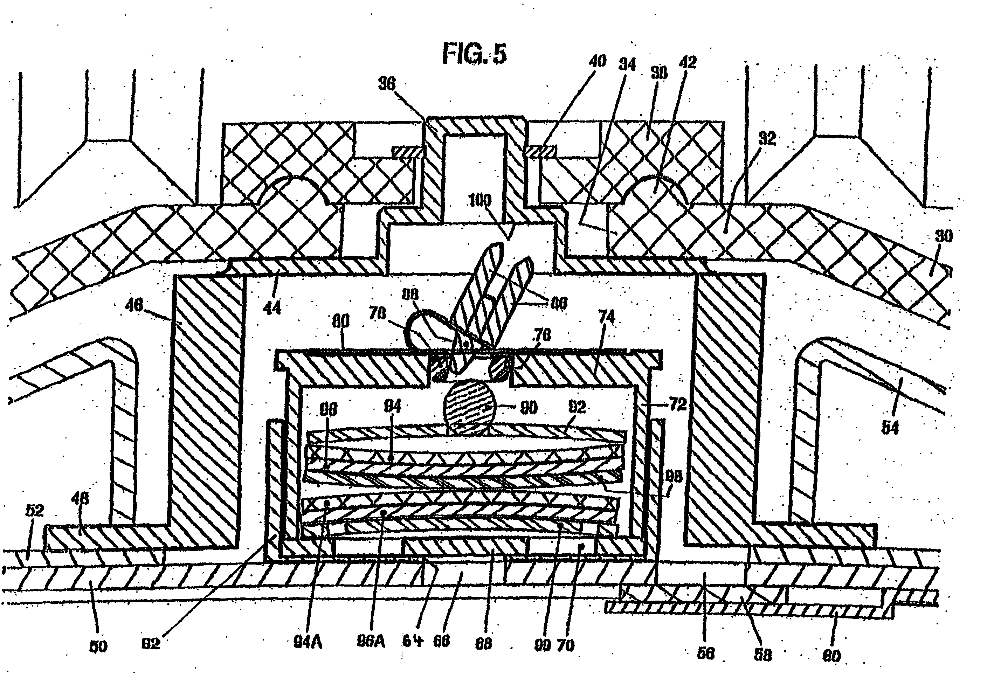

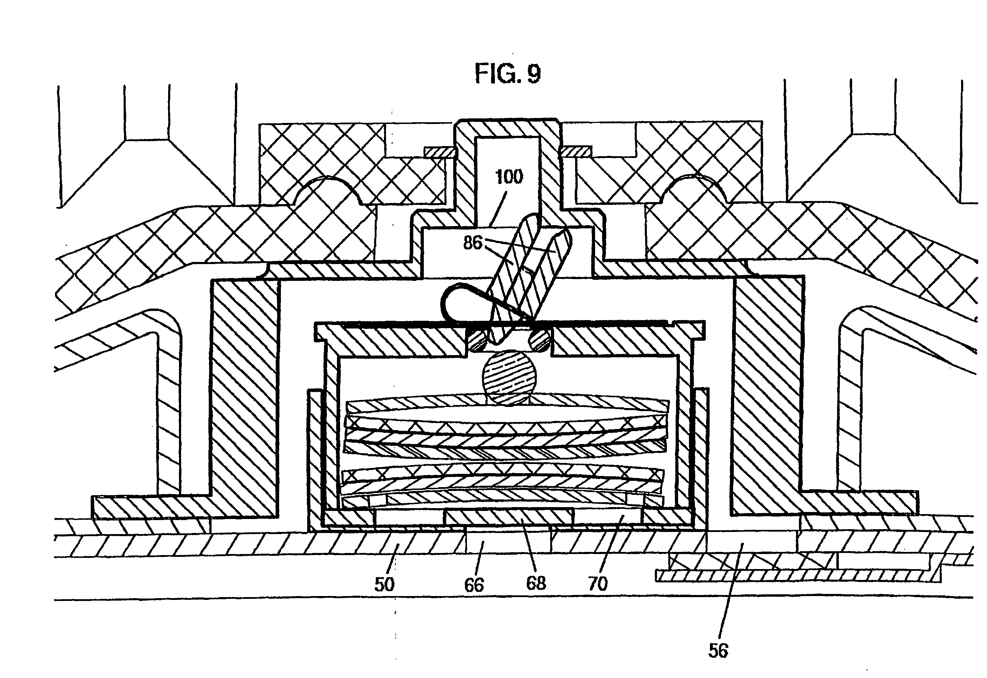

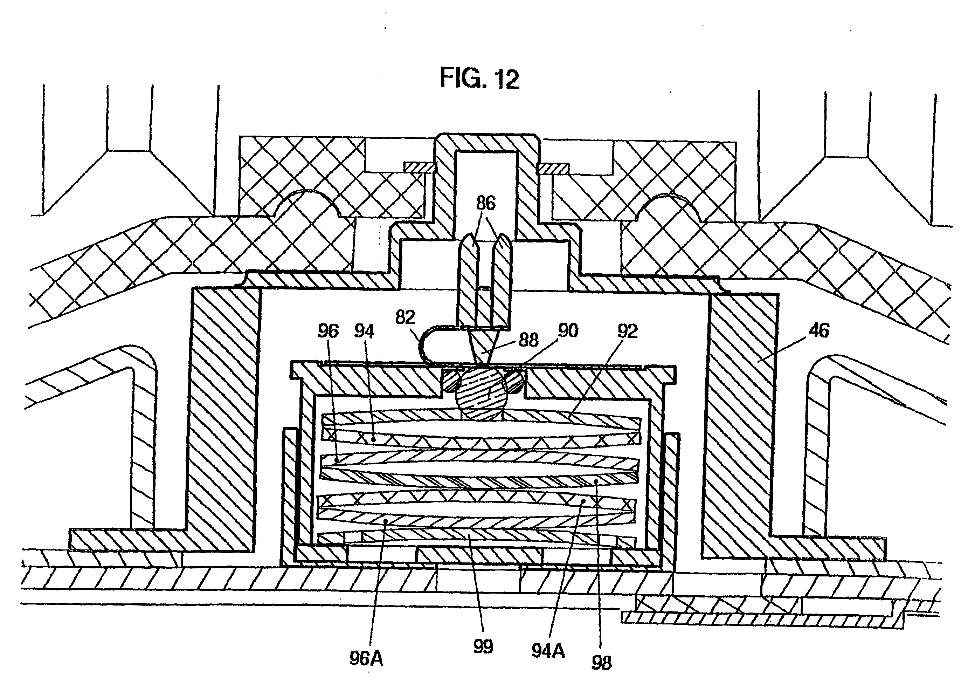

Gemäß der Erfindung sind Vorkehrungen getroffen, um das Ventil auch dann in der Offenstellung zu halten, wenn während des Sterilisiervorganges ein Strömungsdruck auf dem von der Ventilplatte 50 gebildeten Ventilkörper lastet, der das Ventil gegen die Federvorspannung zu schließen trachtet. Die Fig. 8 und 9 lassen erkennen, daß ein auf der Ventilplatte 50 von unten nach oben wirkender Druck die Ventilplatte und die von ihr getragenen Teile nur so weit verschoben hat, bis der durch die Feder 82 in Schrägstellung gehaltene Blockadestift 86 gegen die Stufe 100 des Ventildeckels 36 anschlägt. Diese Anschlagstellung ist aus den Fig. 8 und 9 ersichtlich. Auch in dieser Stellung wird ein Strömungsspalt zwischen dem Ventilsitz 28 und dem Ventilring 26 aufrechterhalten, so daß der Medienaustausch weiter unbehindert vonstatten gehen kann. Es wird daher ein Zuschlagen des Behälters mit Sicherheit verhindert, so daß man beliebige Ladungen in beliebig schnellen Sterilisatoren sterilisieren kann, ohne daß der Behälter zerstört werden kann. Während der Abkühlungsphase schalten bei ca. 95°C die Schnappscheiben 94 und 94A in die Stellung gemäß Fig. 12 um, während die Wölbung der Schnappscheiben 96 und 96A noch unverändert bleibt. Dies ergibt ein Expandieren des Schnappscheibenpakets und damit ein Anheben der Ventilkugel 90, die in dieser Stellung gemäß Fig. 12 dem Dichtungsring 78 anliegt und den Ventilraum von oben her abschließt. Bei der Aufwärtsbewegung der Kugel 90 hat diese den Blockadestift 96 über den Einpreßpin 88 angehoben und, wie aus Fig. 12 ersichtlich, geradegestellt, so daß der Blockadestift frei in den Ventildeckel 36 einlaufen kann. Während des Druckabfalls im Sterilisator nimmt der Außendruck gegenüber dem Innendruck im Behälter und im Ventilraum zunehmend ab, und es erfolgt ein Druckausgleich über das immer noch offene Ventil 26, 28, wobei der Druckausgleich innerhalb der Ventilkammer über das Rückschlagventil 58 bzw. die Entlüftungsöffnung 56 erfolgen kann. Ein weiteres Verdampfen - und damit Vakuumkühlen - innerhalb des Temperatursensors kann jetzt nicht mehr stattfinden, so daß ein frühzeitiges, unerwünschtes Schalten des Sensors zuverlässig verhindert wird.

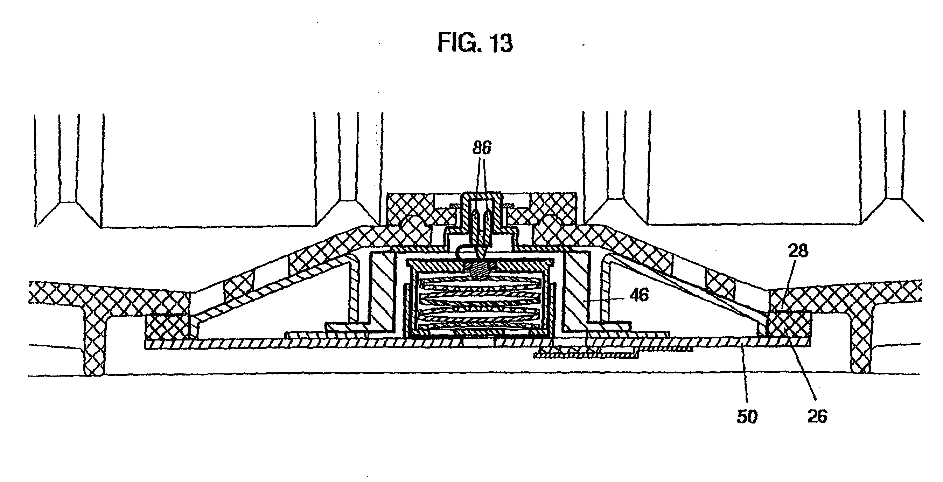

Sobald in der letzten Belüftungsphase der Druck im Sterilisator wieder ansteigt, wird infolge der sich aufbauenden Druckdifferenz der Faltenbalg zunehmend zusammengepreßt und das Ventil 26, 28 wird geschlossen, wobei der Blockadestift 86, wie aus Fig. 13 ersichtlich ist, in das Innere des Ventildeckels 36 einlaufen kann. Bei diesem Vorgang wird der Faltenbalg 46 zusammengepreßt. Diese Schließstellung des Ventils 26, 28 bleibt während der weiteren Abkühlung und auch nach Entnahme des Sterilisationsbehälters aus dem Sterilisator erhalten, weil im Inneren des Sterilisierbehälters ein Unterdruck aufrechterhalten bleibt und der atmosphärische Druck das Ventil geschlossen hält. Durch entsprechende Dimensionierung des Ventilspaltes bzw. des Faltenbalges (Querschnitt und Federkonstante) kann die Höhe des eingeschlossenen Unterdrucks in weiten Grenzen variiert werden.

Bei Abkühlung auf die Schalttemperatur der Schnappscheiben 96, 96A (z.B. 35 bis 50°C) schnappen diese in ihre entgegengesetzte Wölbungsstellung um, wodurch die Ventilkugel 90 von ihrem Sitz abgehoben wird. Der Ventilraum wird dabei über die Belüftungsöffnung 66 belüftet, nicht aber der Behälterinnenraum. Die Ventilanordnung selbst verbleibt in der Schließstellung, bis über ein zusätzliches, in der Zeichnung nicht dargestelltes Belüftungsventil und einen diesem vorgeschalteten Filter der atmosphärische Druck in den Sterilisierbehälter eindringen kann. Dann kehrt infolge der Federwirkung des Faltenbalges 46 und durch Schwerkraft die Ventilanordnung in die Stellung gemäß Fig. 4 und 5 zurück, und der Sterilisierbehälter ist für den nächsten sterilisiervorgang ohne manuelle Manipulation einsetzbar.

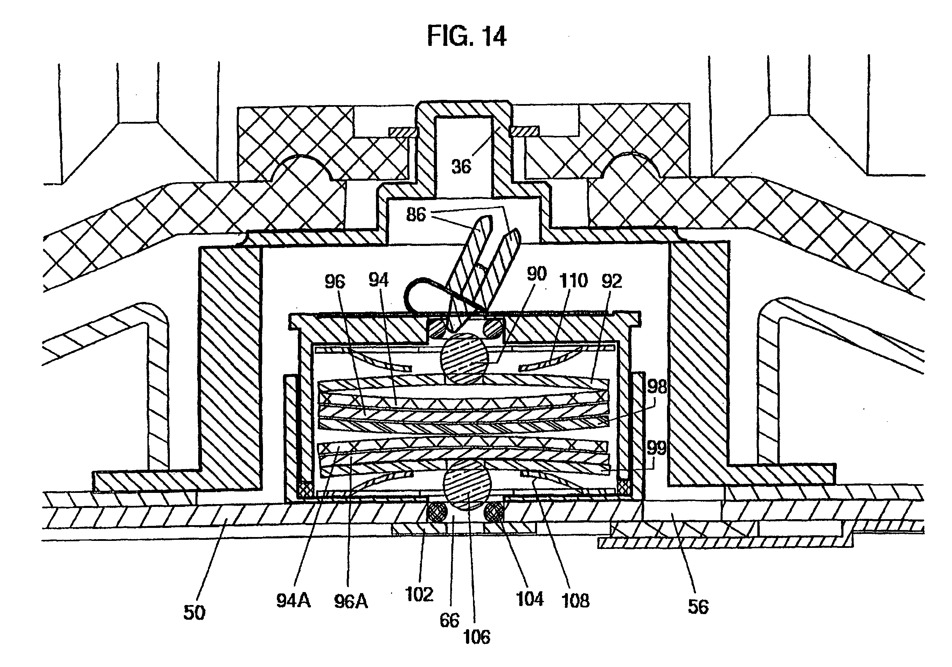

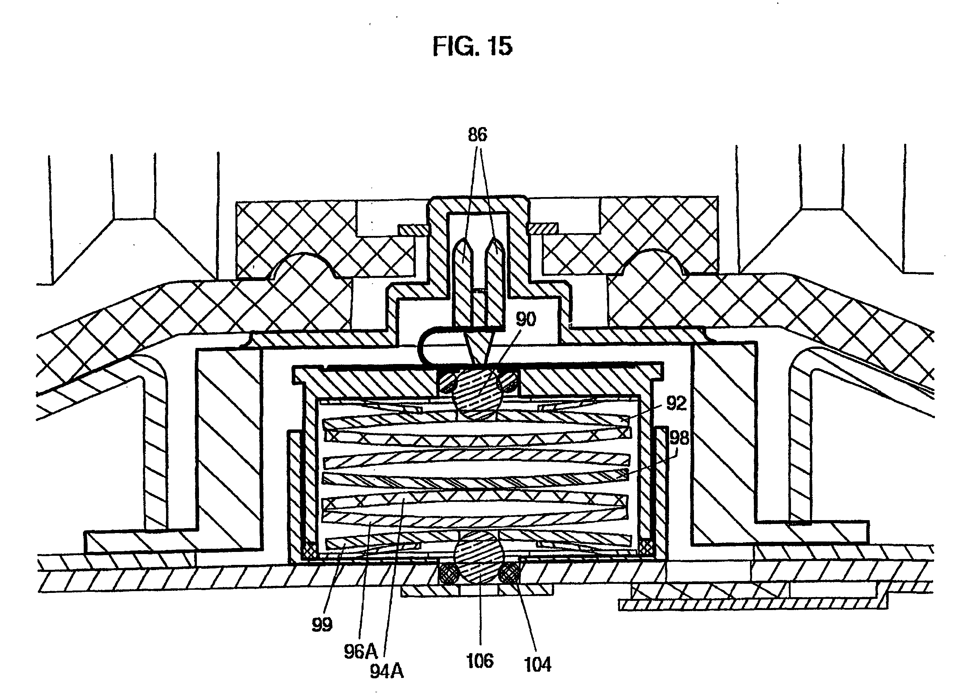

Ein weiteres Ausführungsbeispiel ist in den Fig. 14 und 15 dargestellt. Die Funktion der hier dargestellten Ventilanordnung entspricht im wesentlichen der Funktion bei dem gemäß Fig. 1 bis 13 dargestellten Ausführungsform. Abgewandelt gegenüber dem vorbeschriebenen Ausführungsbeispiel ist die Ventilanordnung insofern, als anstelle der Auslaßdichtungsscheibe 68 ein zweites Kugelventil angeordnet ist. Dieses besteht aus einem in die Belüftungsöffnung 66 eingesetzten und durch eine Scheibe 102 gesicherten Ventilsitzring 104, der mit einer Ventilkugel 106 zusammenwirkt, die mit der konvex gewölbten Edelstahlscheibe 99 verschweißt ist. Diese Scheibe 99 ist durch Federn 108 vorgespannt, wodurch die Ventilkugel 106 von ihrem Sitz abgehoben wird. Weitere Federn 110 wirken auf die obere Edelstahlscheibe 92, wodurch die Ventilkugel 90 in die Öffnungsstellung vorgespannt wird. Hierdurch wird bewirkt, daß die Belüftungsöffnung 66 ständig geöffnet ist, während das Ventil offen ist. Erst nach Umschalten in die Stellung gemäß Fig. 15, die der Stellung nach Fig. 12 entspricht, wird die Belüftungsöffnung geschlossen. Der Unterschied gegenüber dem ersten Ausführungsbeispiel besteht demgemäß darin, daß nach Schalten der Schnappscheiben das Schnappscheibengehäuse komplett in beiden Richtungen abgesperrt ist.

### Bezugszeichenliste

- 10: Wanne
- 12: Deckel
- 14: Gefälleboden
- 16: Standrahmen
- 18: Dichtungsring
- 20: Nut
- 22: Perforationslöcher
- 24: Ventilanordnung
- 26: Dichtring, Ventilring
- 28: Ventilsitz
- 30: konischer Wandabschnitt
- 32: flacher Abschnitt
- 34: Loch
- 36: Ventildeckel
- 38: Drehverschluß
- 40: Seegerring
- 42: Knöpfe
- 44: Ventildeckelflansch
- 46: Well- oder Faltenbalg
- 48: Ringflansch
- 50: Ventilplatte
- 52: Zwischenring
- 54: Leitblech
- 56: Entlüftungsöffnung
- 58: Dichtungsscheibe
- 60: Blattfeder
- 62: Napf
- 64: Mittelöffnung
- 66: Belüftungsöffnung
- 68: Auslaßdichtungsscheibe
- 70: Öffnungen
- 72: Ringwand
- 74: Deckel
- 76: Mittelöffnung
- 78: Dichtungsring
- 80: Federstahlplatte
- 82: Blockadefeder
- 84: Ausschnitt
- 86: Blockadestift
- 88: Einpreßpin
- 90: Ventilkugel
- 92: Edelstahlscheibe
- 94,94A: Schnappscheibe
- 96,96A: Schnappscheibe
- 98: Edelstahlscheibe
- 99: Edelstahlscheibe
- 100: Stufe
- 102: Scheibe
- 104: Ventilsitzring
- 106: Ventilkugel
- 108: Federn
- 110: Federn

## Patentansprüche

1. Sterilisierbehälter (10, 12) mit einer Ventilanordnung (26; 28), die einen Medienaustausch innerhalb eines Sterilisators bis in die Vakuum-Trockenphase hinein ermöglicht und in der letzten Belüftungsphase schließt, so daß der zu diesem Zeitpunkt im Behälterinneren herrschende Unterdruck aufrechterhalten und der Behälter hermetisch verschlossen bleibt, wobei die Ventilanordnung einen dem Strömungsdruck ausgesetzten Ventilkörper (26, 50) und einen Temperatursensor aufweist,
**dadurch gekennzeichnet, daß** am Ventilkörper (26, 50) ein Anschlag (86) vorgesehen ist, der mit einem Gegenanschlag (100) des Behälters zusammenwirkt und den Ventilkörper während der Sterilisierung gegen ein Schließen sperrt, bis die Anschlagverbindung vor oder während der letzten Belüftungsphase durch den Temperatursensor unwirksam gemacht. wird.

2. Sterilisierbehälter nach Anspruch 1,
**dadurch gekennzeichnet, daß** der Temperatursensor gegen eine vorzeitige Abkühlung infolge Kondensat-Rückverdampfung während der Vakuumtrocknung durch eine Abschirmung (62, 74; 46) geschützt ist.

3. Sterilisierbehälter nach Anspruch 1 oder 2, **dadurch gekennzeichnet, daß** der Temperatursensor von einem Schnappscheibenpaket gebildet ist,
wobei zwei Schnappscheibentypen (94, 96) mit unterschiedlichem Temperaturverhalten in einem Schnappscheibenpaket zusammengefaßt sind.

4. Sterilisierbehälter nach einem der Ansprüche 1 bis 3,
**dadurch gekennzeichnet, daß** die Ventilanordnung (26, 28) an einer vertieften Stelle des Behälterbodens zusätzlich als Kon-densatablaßventil angeordnet ist.

5. Sterilisierbehälter nach Anspruch 4,
**dadurch gekennzeichnet, daß** der Behälterboden einen zentralen, konisch nach oben verlaufenden Wandabschnitt (30) mit Perforationslöchern (22) aufweist, und daß der Behälterboden in einem die Perforationslöcher (22) umschließenden Ringabschnitt einen Ventilsitz (28) bildet, der mit einem Ventilkörper in Gestalt eines Ventilringes (26) zusammenwirkt, der von einer Ventilplatte (50) getragen wird.

6. Sterilisierbehälter nach den Ansprüchen 3 und 5,
**dadurch gekennzeichnet, daß** die Ventilplatte (50) in ihrem Mittelabschnitt ein Gehäuse (62, 72, 74) trägt, das das Schnappscheibenpaket (94, 96) aufnimmt, welches eine Ventilkugel (90) steuert, die mit einem Ventilsitzring (78) zusammenwirkt.

7. Sterilisierbehälter nach Anspruch 6,
**dadurch gekennzeichnet, daß** das Schnappscheibengehäuse (62, 72, 74) von einem Faltenbalg (46) umschlossen ist, der im unteren Teil mit der Ventilplatte (50) verbunden ist und mit seinem oberen Abschnitt an einem Ventildeckel (36) festgelegt ist, der seinerseits im Bereich einer Bodenöffnung (34) des Sterilisierbehälters lösbar befestigt ist.

8. Sterilisierbehälter nach Anspruch 7,
**dadurch gekennzeichnet, daß** der napfartig ausgebildete Ventildeckel (36) durch die Bodenöffnung (34) gesteckt und dort lösbar fixiert ist.

9. Sterilisierbehälter nach den Ansprüchen 6 und 7,
**dadurch gekennzeichnet, daß** der Deckel (74) des Schnappscheibengehäuses eine Blockadefeder (82) mit Blockadestift (86) trägt, der mit einem Ventildeckelanschlag (100) als Schließsperre des Ventils (26, 28) zusammenwirkt.

10. Sterilisierbehälter nach Anspruch 9,
**dadurch gekennzeichnet, daß** der Blockadestift (86) durch die Ventilkugel (90) des Schnappscheibenpaketes in die Freigabestellung überführbar ist.

11. Sterilisierbehälter nach einem der Ansprüche 6, 7 und 9,
**dadurch gekennzeichnet, daß** der Boden des Schnappscheibengehäuses eine Auslaßdichtungsscheibe (68) trägt, die über einer Belüftungsöffnung (66) der Ventilplatte (50) als Rückschlagventil anliegt.

12. Sterilisierbehälter nach Anspruch 7,
**dadurch gekennzeichnet, daß** die Ventilplatte in dem vom Faltenbalg (46) umschlossenen Raum eine Entlüftungsöffnung (56) aufweist, die durch eine von einer Blattfeder (60) in Dichtungsstellung vorgespannte Dichtungsscheibe (58) als Rückschlagventil verschließbar ist.

13. Sterilisierbehälter nach Anspruch 11,
**dadurch gekennzeichnet, daß** das Schnappscheibengehäuse einen napfartigen Unterteil (62) mit Innengewinde aufweist, in das eine Ringwand (72) des Deckels (74) einschraubbar ist, die mit ihrer unteren Stirnseite gegen die Dichtungsscheibe (68) wirkt.

14. Sterilisierbehälter nach einem der Ansprüche 6 bis 13,
**dadurch gekennzeichnet, daß** der von der Blockadefeder (82) getragene Blockadestift den Pin (88) trägt, der in den Bewegungsbereich der Ventilkugel (90) eingreift.

15. Sterilisierbehälter nach Anspruch 11,
**dadurch gekennzeichnet, daß** die in das Innere des Schnappscheibengehäuses führende Belüftungsöffnung (66) durch eine Ventilkugel (106) verschließbar ist, die am Boden des Schnappscheibenpaketes angeordnet ist.

16. Sterilisierbehälter nach einem der Ansprüche 1 bis 15,
**dadurch gekennzeichnet, daß** die Ventilplatte (50) ein konisches Leitblech (54) als Kondensatabweiser unter den Perforationslöchern (22) des Behälterbodens aufweist.

17. Sterilisierbehälter nach Anspruch 3,
**dadurch gekennzeichnet, daß** die Schnappscheiben (94, 96) unterschiedlicher Temperaturcharakteristik paarweise angeordnet sind, die bei Raumtemperatur eine gleiche Wölbungskonfiguration aufweisen.

18. Sterilisierbehälter nach Anspruch 17,
**dadurch gekennzeichnet, daß** im Schnappscheibengehäuse zwei Schnappscheibenpaare (94, 96) angeordnet sind, von denen das eine Paar nach oben konkav gewölbt und das andere Paar nach oben konvex gewölbt ist.

19. Sterilisierbehälter nach den Ansprüchen 17 und 18,
**dadurch gekennzeichnet, daß** über dem oberen Schnappscheibenpaar (94, 96) eine nach oben konvex gekrümmte Edelstahlscheibe (92) mit unveränderbarer Krümmung liegt, die im Mittelteil die Ventilkugel (90) trägt.

20. Sterilisierbehälter nach den Ansprüchen 17 bis 19,
**dadurch gekennzeichnet, daß** das untere Schnappscheibenpaar (94A, 96A) durch eine nach oben konvex gewölbte, in ihrer Form unveränderbare Edelstahlscheibe (99) abgestützt ist, und daß zwischen den Schnappscheibenpaaren (94, 96, 94A, 96A) eine nach oben konkav gekrümmte Edelstahlscheibe (98) liegt.

21. Sterilisierbehälter nach einem der Ansprüche 17 bis 20
**dadurch gekennzeichnet, daß** die jeweils obere Schnappscheibe (94, 94A) jedes Schnappscheibenpaares bei Erwärmung einen Umschnapp-Punkt bei z.B. 115°C aufweist und bei Abkühlen unter Hysterese ein Rückschnappen bei einer deutlich niedrigeren Temperatur von z.B. 95°C bewirkt.

22. Sterilisierbehälter nach einem der Ansprüche 17 bis 21,
**dadurch gekennzeichnet, daß** die jeweils untere Schnappscheibe (96, 96A) jedes Schnappscheibenpaares bei Erwärmung einen Umschnapp-Punkt von mehr als etwa 117°C aufweist und bei Abkühlen unter Hysterese ein Rückschnappen erst bei 35 bis 50°C bewirkt.

23. Sterilisierbehälter nach einem der Ansprüche 15 und 17 bis 22,
**dadurch gekennzeichnet, daß** die mit der Belüftungsöffnung (66) zusammenwirkende Ventilkugel (106) von der unteren Edelstahlscheibe (99) getragen wird.

24. Sterilisierbehälter nach einem der Ansprüche 1 bis 23,
**dadurch gekennzeichnet, daß** zwischen einem unteren Ringflansch (48) des Faltenbalgs (46) und der Ventilplatte (50) ein Distanzstück (52) vorgesehen ist.

25. Sterilisierbehälter nach Anspruch 1 oder 2,
**dadurch gekennzeichnet, daß** zwischen Behälterdeckel (12) und Wanne (10) ein im Querschnitt L-förmiger Dichtungsring (18) angeordnet ist, der mit seinem radial nach innen stehenden Schenkel in eine Umfangsnut (20) des Behälterdeckels eingesetzt ist und mit einer radialen und horizontalen Ringfläche des Wannenoberrandes zusammenwirkt.

## Claims

1. Sterilising container (10, 12) having a valve assembly (26; 28) which makes it possible to exchange media within a steriliser up until the vacuum drying phase and shuts in the final aeration phase, thereby maintaining the partial vacuum obtaining at that instant in the interior of the container and keeping the container hermetically sealed, the valve assembly featuring a valve body (26, 50) which is exposed to the streaming pressure, and a temperature sensor,
**characterised in that** provided on the valve body (26, 50) is a catch (86) which cooperates with a mating catch (100) on the container and stops the valve body from shutting during sterilisation until the temperature sensor renders the catch connection inoperative before or during the final aeration phase.

2. Sterilising container according to claim 1,
**characterised in that** during vacuum drying a shield (62, 74; 46) protects the temperature sensor from cooling down prematurely due to re-evaporation of the condensate.

3. Sterilising container according to claim 1 or 2,
**characterised in that** the temperature sensor is constituted by a snap-action disc assembly in which two types of snap-action discs (94, 96) with different thermal behaviour are combined in one snap-action disc assembly.

4. Sterilising container according to any of claims 1 to 3,
**characterised in that** the valve assembly (26, 28) is additionally disposed as a condensate bleed valve, at a recessed point of the container floor.

5. Sterilising container according to claim 4,
**characterised in that** the container floor features a central wall portion (30) with perforation holes (22) which extends conically upwards, and that in an annular portion set around the perforation holes (22) the container floor forms a valve seat (28) which cooperates with a valve body in the form of a valve ring (26) carried by a valve plate (50).

6. Sterilising container according to claims 3 and 5,
**characterised in that** in its middle portion the valve plate (50) carries an enclosure (62, 72, 74) which accommodates the snap-action disc assembly (94, 96) which controls a valve ball (90) which cooperates with a valve seating ring (78).

7. Sterilising container according to claim 6,
**characterised in that** the snap-action disc enclosure (62, 72, 74) is surrounded by a bellows (46) the bottom part of which is joined to the valve plate (50) and which is located by its upper portion on a valve bonnet (36) which in turn is removably affixed in the vicinity of an opening (34) in the floor of the sterilising container.

8. Sterilising container according to claim 7,
**characterised in that** the cup-shaped valve cover (36) is pushed through the opening (34) in the floor and there removably fixed in place.

9. Sterilising container according to claims 6 and 7,
**characterised in that** the cover (74) of the snap-action disc enclosure carries a deadlocking spring (82) with deadlocking pin (86) which cooperates with a valve cover catch (100) to act as a closure catch for the valve (26, 28).

10. Sterilising container according to claim 9,
**characterised in that** the interlocking pin (86) is adapted to be moved into the release position by the valve ball (90) of the snap-action disc assembly.

11. Sterilising container according to any of claims 6, 7 and 9,
**characterised in that** the floor of the snap-action disc enclosure carries a discharge sealing gasket (68) which as a return valve rests over a ventilation opening (66) in the valve plate (50).

12. Sterilising container according to claim 7,
**characterised in that** in the compartment surrounded by the bellows (46) the valve plate features a vent hole (56) which can be sealed by a gasket (58) to act as a return valve, which hole is tensioned in the sealing position by a leaf spring (60).

13. Sterilising container according to claim 11,
**characterised in that** the snap-action disc enclosure features an internally threaded, cup-shaped bottom section (62) into which one annular wall (72) of the cover (74) can be screwed, the lower end face of said wall operating against the gasket (68).

14. Sterilising container according to any of claims 6 to 13, **characterised in that** the interlocking pin carried by the interlocking spring (82) carries the peg (88) which interferes in the range of travel of the valve ball (90).

15. Sterilising container according to claim 11,
**characterised in that** the ventilation opening (66) leading into the interior of the snap-action disc enclosure can be sealed by a valve ball (106) disposed on the floor of the snap-action disc assembly.

16. Sterilising container according to any of claims 1 to 15,
**characterised in that** the valve plate (50) features a conical baffle (54) to act as a condensate deflector beneath the perforation holes (22) set in the container floor.

17. Sterilising container according to claim 3,
**characterised in that** the snap-action discs (94, 96) with their various temperature characteristics are arranged in pairs configured so as to exhibit the same convexity at room temperature.

18. Sterilising container according to claim 17,
**characterised in that** arranged in the snap-action disc enclosure are two pairs of snap-action discs (94, 96), one pair of which has upwardly concave curvature and the other pair upwardly convex curvature.

19. Sterilising container according to claims 17 and 18,
**characterised in that** situated above the upper pair of snap-action discs (94, 96) is a stainless-steel disc (92) having invariable, upwardly convex curvature, the middle portion of which carries the valve ball (90).

20. Sterilising container according to claims 17 to 19,
**characterised in that** the lower pair of snap-action discs (94A, 96A) is supported by a stainless-steel disc (99) with upwardly convex curvature and an invariable shape, and that situated between the pairs of snap-action discs (94, 96, 94A, 96A) is a stainless-steel disc (98) with upwardly concave curvature.

21. Sterilising container according to any of claims 17 to 20,
**characterised in that** the respective upper snap-action disc (94, 94A) of each pair of snap-action discs has a snapping point of, say, 115°C upon being heated, and brings about a snapping-back action at a markedly lower temperature of, say, 95°C upon being cooled, with attendant hysteresis.

22. Sterilising container according to any of claims 17 to 21,
**characterised in that** the respective lower snap-action disc (96, 96A) of each pair of snap-action discs has a snapping point of above approx. 117°C upon being heated, and only snaps back at around 35 to 50 °C upon being cooled, with attendant hysteresis.

23. Sterilising container according to any of claims 15 and 17 to 22,
**characterised in that** the valve ball (106) which cooperates with the ventilation opening (66) is carried by the lower stainless-steel disc (99).

24. Sterilising container according to any of claims 1 to 23,
**characterised in that** a spacer (52) is provided between a lower annular flange (48) of the bellows (46) and the valve plate (50).

25. Sterilising container according to claim 1 or 2,
**characterised in that** disposed between the container cover (12) and the tank (10) is a gasket (18) of L-shaped cross-section which is inserted by its radially inward sidepiece into a circumferential groove (20) in the container cover and cooperates with a radial and horizontal annular surface at the top edge of the tank.

## Revendications

1. Récipient de stérilisation (10, 12) ayant un dispositif de ventilation (26 ; 28), qui permet un échange de milieux au sein d'un stérilisateur jusqu'à la phase de séchage à vide et se ferme dans la dernière phase de ventilation, de telle sorte que la dépression régnant à l'intérieur du récipient à ce moment est conservée et le récipient reste fermé hermétiquement, moyennant quoi, le dispositif de ventilation comporte un corps de soupape(26; 28) exposé à la pression d'écoulement et un capteur de température, **caractérisé en ce qu'**une butée (86) est prévue sur le corps de soupape (26, 50) qui coopère avec une butée opposée (100) du récipient et bloque le corps de soupape pendant la stérilisation contre une fermeture jusqu'à ce que la liaison de butée avant ou pendant la dernière phase de ventilation soit rendue inefficace par le capteur de température.

2. Récipient de stérilisation selon la revendication 1, **caractérisé en ce que** le capteur de température est protégé par un blindage (62, 74 ; 46) contre un refroidissement prématuré à la suite de l'évaporation en retour du condensat pendant le séchage à vide.

3. Récipient de stérilisation selon la revendication 1 ou 2, **caractérisé en ce que** le capteur de température est formé par un paquet de disques à enclenchement, deux types de disques à enclenchement (94, 96) avec des comportements en température différents étant assemblés en un paquet de disques à enclenchement.

4. Récipient de stérilisation selon l'une quelconque des revendications 1 à 3, **caractérisé en ce que** le dispositif de ventilation (26, 28) est disposé à un endroit profond du fond du récipient, en outre, en tant que soupape de décharge de condensat.

5. Récipient de stérilisation selon la revendication 4, **caractérisé en ce que** le fond du récipient comporte une section de paroi (30) centrale, conique s'étendant vers le haut, avec des trous de perforation (22), et **en ce que** le fond du récipient forme un siège de soupape (28) dans une section annulaire entourant les trous de perforation (22), lequel siège coopère avec un corps de soupape sous forme d'un anneau de soupape (26) qui est porté par une plaque porte-soupape (50).

6. Récipient de stérilisation selon les revendications 3 et 5, **caractérisé en ce que** la plaque porte-soupape (50) supporte dans sa section médiane un carter (62, 72, 74) qui reçoit le paquet de disques à enclenchement (94, 96) qui commande une bille de soupape (90) qui coopère avec un anneau de siège de soupape (78).

7. Récipient de stérilisation selon la revendication 6, **caractérisé en ce que** le carter de disques à enclenchement (62, 72, 74) est entouré d'un soufflet d'intercirculation (46), qui est relié à la partie inférieure de la plaque porte-soupape (50) et est fixé par sa section supérieure à un couvercle de soupape (36) qui de son côté est fixé de manière amovible au niveau d'un orifice de fond (34) du récipient de stérilisation.

8. Récipient de stérilisation selon la revendication 7, **caractérisé en ce que** le couvercle de soupape (36) formé comme une cuvette est placé à travers l'orifice du fond et y est fixé de manière amovible.

9. Récipient de stérilisation selon les revendications 6 et 7, **caractérisé en ce que** le couvercle (74) du carter de disques à enclenchement porte un ressort de blocage (82) avec une tige de blocage (86) qui coopère avec une butée (100) du couvercle de soupape en tant que dispositif de blocage de fermeture de la soupape (26, 28).

10. Récipient de stérilisation selon la revendication 9, **caractérisé en ce qu'**il est possible de faire passer la tige de blocage (86) à la position de déblocage par la bille de soupape (90) du paquet de disques à enclenchement.

11. Récipient de stérilisation selon l'une quelconque des revendications 6, 7 et 9, **caractérisé en ce que** le fond du carter de disques à enclenchement porte une rondelle d'étanchéité d'évacuation (68) qui adhère uniformément sur un orifice de ventilation (66) de la plaque porte-soupape (50) en tant que soupape de retenue.

12. Récipient de stérilisation selon la revendication 7, **caractérisé en ce que** la plaque porte-soupape comporte un évent (56) dans l'espace entouré par le soufflet d'intercirculation (46) qui peut être obturé par une rondelle d'étanchéité (58) précontrainte par un ressort à lames (60) dans la position étanche en tant que soupape de retenue.

13. Récipient de stérilisation selon la revendication 11, **caractérisé en ce que** le carter de disques à enclenchement comporte une partie inférieure (62) en forme de cuvette avec un taraudage, dans lequel une paroi circulaire (72) du couvercle (74) peut être vissée, laquelle agit avec son côté avant inférieur contre la rondelle d'étanchéité (68).

14. Récipient de stérilisation selon l'une quelconque des revendications 6 à 13, **caractérisé en ce que** la tige de blocage soutenue par le ressort de blocage (82) supporte la broche (88) qui s'engage dans la zone de mouvement de la bille de soupape (90).

15. Récipient de stérilisation selon la revendication 11, **caractérisé en ce que** l'orifice de ventilation (66) menant à l'intérieur du carter de disques à enclenchement peut être obturé par une bille de soupape (106) qui est disposée au fond du paquet de disques à enclenchement.

16. Récipient de stérilisation selon l'une quelconque des revendications 1 à 15, **caractérisé en ce que** la plaque porte-soupape (50) comporte une tôle de guidage conique (54) en tant que déflecteur de condensat sous les orifices de perforation (22) du fond du récipient.

17. Récipient de stérilisation selon la revendication 3, **caractérisé en ce que** les disques à enclenchement (94, 96) ayant des caractéristiques de température différentes sont disposés par paires qui présentent à température ambiante une configuration de voûte identique.

18. Récipient de stérilisation selon la revendication 17, **caractérisé en ce que** deux paires de disques à enclenchement (94, 96) sont disposés dans le carter de disques à enclenchement, dont l'une des paires est bombée de manière concave vers le haut et l'autre paire bombée de manière convexe vers le haut.

19. Récipient de stérilisation selon les revendications 17 et 18, **caractérisé en ce que** au-dessus de la paire supérieure de disques à enclenchement se trouve un disque en acier fin (92) courbé vers le haut de manière convexe ayant une courbure qui ne peut être modifiée, laquelle courbure supporte la bille de soupape (90) dans la partie centrale.

20. Récipient de stérilisation selon les revendications 17 à 19, **caractérisé en ce que** la paire inférieure de disques à enclenchement (94A, 96A) est supportée par un disque en acier fin (99) non modifiable dans sa forme, bombé de manière convexe vers le haut, et **en ce que** entre les paires de disques à enclenchement (94, 96, 94A, 96A) se trouve un disque en acier fin (98) courbé de manière concave vers le haut.

21. Récipient de stérilisation selon l'une quelconque des revendications 17 à 20, **caractérisé en ce que** le disque à enclenchement respectif supérieur (94, 94A) de chaque paire de disques à enclenchement comporte en cas de réchauffement un point d'inversion à environ 115°C et en cas de refroidissement sous l'hystérèse un réenclenchement se produit à une température nettement inférieure d'environ 95°C.

22. Récipient de stérilisation selon l'une quelconque des revendications 17 à 21, **caractérisé en ce que** le disque à enclenchement respectif inférieur (96, 96A) de chaque paire de disques à enclenchement comporte en cas de réchauffement un point d'inversion, supérieur à environ 117°C et en cas de refroidissement sous l'hystérèse un réenclenchement ne se produit qu'à une température située entre 35 et 50°C.

23. Récipient de stérilisation selon l'une quelconque des revendications 15 et 17 à 22, **caractérisé en ce que** la bille de soupape (106) coopérant avec l'orifice de ventilation (66) est supportée par le disque en acier fin inférieur (99).

24. Récipient de stérilisation selon l'une quelconque des revendications 1 à 23, **caractérisé en ce que** entre une bride annulaire inférieure (48) du soufflet d'intercirculation (46) et la plaque porte-soupape (50) une pièce d'écartement est prévue.

25. Récipient de stérilisation selon la revendication 1 ou 2, **caractérisé en ce que** entre le couvercle de récipient (12) et la cuve (10) est disposé un anneau d'étanchéité (18), en section transversale en forme de L, lequel est monté avec son aile dirigée radialement vers l'intérieur dans une rainure circonférentielle (20) du couvercle de récipient et coopère avec une surface annulaire radiale et horizontale du bord supérieur de la cuve.
